# EUROPEAN PATENT SPECIFICATION

(11) **EP 3 304 086 B1**
(45) Date of publication and mention of the grant of the patent: **23.12.2020**
(21) Application number: 16802790.2
(22) Date of filing: 27.05.2016
(51) Int. Cl.: G01N 33/574, A61P 13/12, A61P 35/00

(54) **METHOD FOR PREDICTING RESPONSIVENESS TO A COMBINATION THERAPY WITH LENVATINIB AND EVEROLIMUS**
METHODE ZUR VORHERSAGE DES ANSPRECHENS AUF EINE KOMBINATIONSTHERAPIE MIT LENVATINIB UND EVEROLIMUS
MÉTHODE POUR PRÉDIRE LA RÉACTIVITÉ À UNE THÉRAPIE COMBINÉE AVEC DU LENVATINIB ET DE L'ÉVÉROLIMUS

(30) Priority: 29.05.2015 US 201562167964 P
(43) Date of publication of application: 11.04.2018
(73) Proprietor: Eisai R&D Management Co., Ltd., Tokyo 112-8088 (JP)
(72) Inventor: FUNAHASHI, Yasuhiro, Tsukuba-shi Ibaraki 300-2635 (JP); KADOWAKI, Tadashi, Tsukuba-shi Ibaraki 300-2635 (JP)
(74) Representative: Hoffmann Eitle
(86) International application number: PCT/JP2016/002562
(87) International publication number: WO 2016/194348

(56) References cited:
- EP-A1- 2 293 071
- WO-A2-2012/166899
- JP-A- 2014 521 308
- H. GLEN ET AL: "432 Correlative analyses of serum biomarkers and clinical outcomes in the phase 2 study of lenvatinib, everolimus, and the combination, in patients with metastatic renal cell carcinoma following 1 VEGF-targeted therapy", EUROPEAN JOURNAL OF CANCER, vol. 51, 1 September 2015 (2015-09-01), page S89, XP055510094, AMSTERDAM, NL ISSN: 0959-8049, DOI: 10.1016/S0959-8049(16)30266-0
- BISHOY A GAYED ET AL: "Prospective evaluation of plasma levels of ANGPT2, TuM2PK, and VEGF in patients with renal cell carcinoma", BMC UROLOGY, BIOMED CENTRAL, LONDON, GB, vol. 15, no. 1, 3 April 2015 (2015-04-03), page 24, XP021217372, ISSN: 1471-2490, DOI: 10.1186/S12894-015-0019-4
- XIAOEN WANG ET AL: "The Role of Angiopoietins as Potential Therapeutic Targets in Renal Cell Carcinoma", TRANSLATIONAL ONCOLOGY, vol. 7, no. 2, 1 April 2014 (2014-04-01), pages 188-195, XP055218621, United States ISSN: 1936-5233, DOI: 10.1016/j.tranon.2014.02.003
- MOLINA ANA M ET AL: "A phase 1b clinical trial of the multi-targeted tyrosine kinase inhibitor lenvatinib (E7080) in combination with everolimus for treatment of metastatic renal cell carcinoma (RCC)", CANCER CHEMOTHERAPY AND PHARMACOLOGY, SPRINGER VERLAG, BERLIN, vol. 73, no. 1, 5 November 2013 (2013-11-05), pages 181-189, XP035339586, ISSN: 0344-5704, DOI: 10.1007/S00280-013-2339-Y [retrieved on 2013-11-05]
- CHING CHING LEOW ET AL: "MEDI3617, a human anti-angiopoietin 2 monoclonal antibody, inhibits angiogenesis and tumor growth in human tumor xenograft models", INTERNATIONAL JOURNAL OF ONCOLOGY, DEMETRIOS A. SPANDIDOS ED. & PUB, GR , vol. 40, no. 5 1 May 2012 (2012-05-01), pages 1321-1330, XP002721374, ISSN: 1019-6439, DOI: 10.3892/IJO.2012.1366 Retrieved from the Internet: URL:http://www.spandidos-publications.com/ 10.3892/ijo.2012.1366 [retrieved on 2012-02-10]
- MOTZER, R. ET AL.: 'RANDOMIZED PHASE II, THREE-ARM TRIAL OF LENVATINIB (LEN), EVEROLIMUS (EVE), AND LEN+EVE IN PATIENTS PTS) WITH METASTATIC RENAL CELL CARCINOMA (MRCC)' JOURNAL OF CLINICAL ONCOLOGY vol. 33, no. 15S, 20 May 2015, page 248S, XP009507694
- MOTZER, R.J. ET AL.: 'Investigation of novel circulating proteins, germ line single-nucleotide polymorphisms, and molecular tumor markers as potential efficacy biomarkers of first-line sunitinib therapy for advanceed renal cell carcinoma' CANCER CHEMOTHERAPY AND PHARMACOLOGY vol. 74, no. 4, 07 August 2014, pages 739 - 750, XP035397550
- NAKAZAWA, Y. ET AL.: 'Multitargeting strategy using lenvatinib and golvatinib: Maximizing anti-angiogenesis activity in a preclinical cancer model' CANCER SCIENCE vol. 106, no. 2, 04 February 2015, pages 201 - 207, XP055332035
- FINN RICHARD S ET AL: "Phase I study investigating everolimus combined with sorafenib in patients with advanced hepatocellular carcinoma", JOURNAL OF HEPATOLOGY, ELSEVIER, AMSTERDAM, NL, vol. 59, no. 6, 6 August 2013 (2013-08-06) , pages 1271-1277, XP028775460, ISSN: 0168-8278, DOI: 10.1016/J.JHEP.2013.07.029

## Description

### [Technical Field]

The present invention relates generally to biomarkers and renal cell carcinoma.

### [Background Art]

A number of kinase inhibitors have been developed as antitumor agents. For example, a group of compounds having inhibitory activity against receptor tyrosine kinases, such as vascular endothelial growth factor receptor (VEGFR), are known to inhibit angiogenesis and are regarded as a new class of antitumor agents. Lenvatinib mesylate (also known as E7080) is an oral tyrosine kinase inhibitor targeting VEGFR1-3, fibroblast growth factor receptor (FGFR) 1-4, rearranged during transfection receptor (RET), KIT, and platelet-derived growth factor receptor (PDGFR). Lenvatinib mesylate has been approved as LENVIMA™ by U.S. Food and Drug Administration for the treatment of patients with locally recurrent or metastatic, progressive, radioactive iodine-refractory differentiated thyroid cancer. Phase 2 and 3 studies for other tumor types, including renal cell carcinoma, hepatocellular carcinoma, endometrial cancer and non-small cell lung cancer, are ongoing.

Unfortunately, most anti-tumor treatments are associated with undesirable side effects, such as profound nausea, vomiting, or severe fatigue. Such side effects need to be controlled especially when multiple anti-tumor agents are used as a combination therapy. Also, while anti-tumor treatments have been successful, they do not produce significant clinical responses in all patients who receive them, resulting in undesirable side effects, delays, and costs associated with ineffective treatment. Therefore, biomarkers that can be used to predict the response of a subject to an antitumor agent, especially when it is used in a combination therapy, prior to administration thereof are greatly needed.

WO 2012/157672 discloses that, in a sub-group of melanoma patients who either have wild type B-raf and PTEN or mutated B-raf and PTEN, high levels of Ang2, IL6, CXCR4, COL4A3, MEIS1, FGF9, FGFR1, FGFR2, FGFR3, FGFR4, or VEGFR1 and low levels of SHC1, NRP2, ARHGAP22, SCG2, or PML are predictive of responsiveness to lenvatinib compounds.

WO 2012/166899 discloses that, in subjects having thyroid or kidney cancer, low levels of Ang2, VEGFA, IFNG, or soluble KDR or high levels of IL-6, IL-13, PDGFAB, CSF3, CCL3, CCL4, FLT4, or FGF2 are predictive of responsiveness to lenvatinib compounds. However, WO 2012/166899 does not disclose or suggest that Ang2 can be used as predictive of the preference for the combination therapy comprising lenvatinib compounds and another anti-tumor agent, such as everolimus, over the monotherapy with such another anti-tumor agent in kidney cancer patients.

WO 2014/185540 discloses that, in subjects having endometrial cancer, low levels of Ang2 is predictive of responsiveness to lenvatinib compounds. However, WO 2014/185540 does not disclose that Ang2 can be used as predictive of responsiveness to the combination therapy comprising lenvatinib compounds and everolimus in renal cell carcinoma patients, much less the preference for such combination therapy over the monotherapy with everolimus.

US 2012/0077837 discloses a method of treating tumor comprising administrating to a patient lenvatinib compound (e.g. lenvatinib mesylate) and everolimus. However, US 2012/0077837 does not disclose or suggest that Ang2 can be used as predictive of responsiveness to the combination therapy comprising lenvatinib compound (e.g. lenvatinib mesylate) and everolimus in renal cell carcinoma patients, much less the preference for such combination therapy over the monotherapy with everolimus.

Gayed et al. BMC Urology, 15(24): 1-8 (2015), reports the prospective evaluation of plasma levels of ANGPT2, TuM2PK, and VEGF in patients with renal cell carcinoma.

Wang et al. Translational Oncology, 7(2): 188-195 (2014), reports the role of angiopoietins as potential therapeutic targets in renal cell carcinoma.

Molina et al. Cancer Chemother Pharmacol, 73:181-189 (2014), reports a phase 1b clinical trial of lenvatinib in combination with everolimus for treatment of metastatic renal cell carcinoma.

Leow et al. International Journal of Oncology, 40 :1321-1330 (2012), reports that MEDI3617 (a human anti-angiopoietin 2 monoclonal antibody) inhibits angiogenesis and tumor growth in human tumor xenograft models.

EP2293071 discloses methods for predicting the clinical outcome or determining the treatment course in a subject afflicted with colorectal cancer and for monitoring the progression of colorectal cancer in a subject.

Llovet et al. Clin. Cancer Res., 18(8):2290-2300 (2012), reports that in patients with advanced hepatocellular carcinoma, while the angiogenesis biomarkers Ang2 and VEGF were predictors of survival, these biomarkers were not predictive of responsiveness to the angiogenesis inhibitor, sorafenib.

Thus, an angiogenesis biomarker like Ang2 is not expected to serve as a biomarker for responsiveness to angiogenesis inhibitors in all cancers.

### [Summary of Invention]

The present application is based, at least in part, on the identification of biomarkers that can be used to identify or select a human subject having, suspected of having, or at risk of developing, a renal cell carcinoma in need of a combination therapy comprising lenvatinib or a pharmaceutically acceptable salt thereof (e.g., lenvatinib mesylate) and everolimus. The expression level of angiopoietin-2 ("Ang2") in a biological sample obtained from the human subject prior to treatment ("baseline level") is identified as a useful predictor of a human subject having, suspected of having, or at risk of developing, a renal cell carcinoma in need of a combination therapy comprising lenvatinib or a pharmaceutically acceptable salt thereof (e.g., lenvatinib mesylate) and everolimus. Thus, the biomarkers and compositions described herein are useful, for example, in identifying, stratifying, and/or selecting a patient or a subset of patients having renal cell carcinoma that could benefit from a combination therapy comprising lenvatinib or a pharmaceutically acceptable salt thereof (e.g., lenvatinib mesylate) and everolimus. In addition, the methods described herein are useful, for example, in selecting appropriate treatment modalities (e.g., combination therapy comprising lenvatinib or a pharmaceutically acceptable salt thereof (e.g., lenvatinib mesylate) and everolimus or an alternative renal cell carcinoma therapy) for a subject suffering from, suspected of having, or at risk of developing a renal cell carcinoma.

In one aspect, the disclosure provides a method of identifying a human subject having, suspected of having, or at risk of developing, a renal cell carcinoma in need of a combination therapy comprising lenvatinib or a pharmaceutically acceptable salt thereof and everolimus. The method involves assaying a biological sample obtained from the subject before administration of the combination therapy and determining that the concentration of Ang2 protein in the biological sample is high, as compared to a control. The subject having a high concentration of Ang2 protein in the biological sample is identified as in need of the combination therapy comprising lenvatinib or a pharmaceutically acceptable salt thereof and everolimus.

In a second aspect, the disclosure features a method of selecting a human subject having, suspected of having, or at risk of developing, a renal cell carcinoma for administration with a combination therapy comprising lenvatinib or a pharmaceutically acceptable salt thereof and everolimus. The method comprises assaying a biological sample obtained from the human subject for the baseline level of Ang2 protein. If it is determined that the baseline level of Ang2 protein in the biological sample is high, as compared to a control, the subject is selected for administration of the combination therapy. In certain aspects, the method further comprises administering the combination therapy to the human subject.

In a third aspect, the disclosure provides a method of treating a renal cell carcinoma. The method involves providing a biological sample obtained from a subject having renal cell carcinoma before the treatment; measuring, in the biological sample, an Ang2 protein expression level that is high as compared to a control; and administering to the subject a therapeutically effective amount of lenvatinib or a pharmaceutically acceptable salt thereof and everolimus.

In a fourth aspect, the disclosure provides a method of treating a renal cell carcinoma. The method involves administering to the subject that has a renal cell carcinoma a therapeutically effective amount of lenvatinib or a pharmaceutically acceptable salt thereof and everolimus, wherein the subject has been identified as having an Ang2 protein expression level that is high as compared to a control. In certain embodiments, the subject has been identified as having a high concentration of Ang2 protein in a biological sample obtained from the human subject.

In a fifth aspect, the disclosure features a method of treating a human subject having, suspected of having, or at risk of developing, a renal cell carcinoma. The method involves administering the human subject with a combination therapy comprising everolimus and lenvatinib or a pharmaceutically acceptable salt thereof, wherein the human subject has been previously determined to have a baseline level of Ang2 protein in a biological sample obtained from the subject that is higher than a control.

The following embodiments are envisaged for all of the above aspects.

In one embodiment the lenvatinib or a pharmaceutically acceptable salt thereof is lenvatinib mesylate.

In one embodiment, the renal cell carcinoma is an advanced renal cell carcinoma or a metastatic renal cell carcinoma.

In some embodiments, the biological sample is selected from the group consisting of a blood sample, a serum sample, a plasma sample, a renal cell carcinoma archived tumor sample, and a renal cell carcinoma biopsy sample.

In some embodiments, the control is a pre-established cut-off value. In one embodiment, the pre-established cut-off value is an Ang2 protein concentration that is determined based on receiver operating characteristic (ROC) analysis or percentile analysis predicting tumor response with a higher positive predictive value compared to no cut-off, and wherein a concentration of Ang2 protein equal to or below the pre-established cut-off value is a low concentration of Ang2 and a value higher than the pre-established cut-off value is a high concentration of Ang2. The tumor response is an objective response rate (ORR), a clinical benefit rate (CBR), or % of maximum tumor shrinkage. In another embodiment, the pre-established cut-off value is an Ang2 protein concentration that is determined based on simulation models or percentile analysis predicting survival, and wherein a concentration of Ang2 protein equal to or below the pre-established cut-off value is a low concentration of Ang2 and a value higher than the pre-established cut-off value is a high concentration of Ang2. In this context, survival is progression free survival (PFS) or overall survival (OS). In a particular embodiment, the pre-established cut-off value is an Ang2 protein concentration that is within the range of 3565 to 5650 (e.g., 3760 pg/ml), and wherein a concentration of Ang2 protein equal to or below the pre-established cut-off value is a low concentration of Ang2 and a value higher than the pre-established cut-off value is a high concentration of Ang2.

In some aspects, the method further includes communicating the test results to the subject's health care provider. In certain aspects, the method further includes modifying the subject's medical record to indicate that the subject is in need of or not in need of a combination therapy comprising lenvatinib or a pharmaceutically acceptable salt thereof and everolimus. In specific aspects, the record is created on a computer readable medium. In certain aspects, the method further includes prescribing a combination therapy comprising lenvatinib or a pharmaceutically acceptable salt thereof and everolimus for the subject if the baseline Ang2 expression profile is predictive that the subject is in need of a combination therapy comprising lenvatinib or a pharmaceutically acceptable salt thereof. In certain aspects, the method further includes prescribing a therapy not comprising lenvatinib or a pharmaceutically acceptable salt thereof and everolimus for the subject if the baseline Ang2 expression profile is predictive that the subject is not in need of a combination therapy comprising lenvatinib or a pharmaceutically acceptable salt thereof and everolimus. In some aspects, the method further includes administering to the subject a combination therapy comprising lenvatinib or a pharmaceutically acceptable salt thereof and everolimus if the baseline Ang2 expression profile is predictive that the subject is in need of a therapy comprising lenvatinib or a pharmaceutically acceptable salt thereof and everolimus. In some aspects, the method further includes administering to the subject a therapy that does not comprise lenvatinib or a pharmaceutically acceptable salt thereof and everolimus if the baseline Ang2 expression profile is predictive that the subject is not in need of a combination therapy comprising lenvatinib or a pharmaceutically acceptable salt thereof and everolimus.

In one embodiment, the concentration of Ang2 is measured by an immunological method. In some embodiments, the immunological method is selected from the group consisting of enzyme immunoassay, radioimmunoassay, chemiluminescent immunoassay, electrochemiluminescence immunoassay, latex turbidimetric immunoassay, latex photometric immunoassay, immuno-chromatographic assay, and western blotting. In another embodiment, the concentration of Ang2 is measured by enzyme immunoassay.

In a sixth aspect, this disclosure provides lenvatinib or a pharmaceutically acceptable salt thereof for concomitant use with everolimus in treating a renal cell carcinoma in a human subject, wherein the human subject is identified by the methods described above as a subject that is in need of a combination therapy comprising lenvatinib or a pharmaceutically acceptable salt thereof and everolimus. In some embodiments, the pharmaceutically acceptable salt of lenvatinib is lenvatinib mesylate. In one embodiment, the renal cell carcinoma is an advanced or metastatic renal cell carcinoma.

In a seventh aspect, the disclosure provides an Ang2 protein detection agent for use in predicting that a human subject having, suspected of having, or at risk of developing, a renal cell carcinoma is in need of a combination therapy comprising lenvatinib or a pharmaceutically acceptable salt thereof and everolimus. In one aspect, the Ang2 protein detection agent is an anti-Ang2 antibody.

In an eighth aspect, the disclosure features a kit comprising an Ang2 protein detection agent for use in predicting that a human subject having, suspected of having, or at risk of developing, a renal cell carcinoma is in need of a combination therapy comprising lenvatinib or a pharmaceutically acceptable salt thereof and everolimus. In certain aspects, the Ang2 protein detection agent is an anti-Ang2 antibody. In certain aspects, the anti-Ang2 antibody is a monoclonal antibody. In other aspects, the anti-Ang2 antibody is a polyclonal antibody. In certain aspects, the antibody is conjugated with a detectable agent. In one aspect, the detectable agent is horse radish peroxidase, biotin, a fluorescent moiety, a radioactive moiety, a histidine tag, or a peptide tag. In one aspect, the detectably labeled antibody is coated on a microplate. In certain aspects, the microplate is a 96 well microplate. In certain aspects, the kit optionally includes one or more concentration standards, one or more buffers (e.g., wash buffers), one or more diluents (e.g., assay and/or calibration diluents), and one or more reagents that facilitate detecting whether the Ang2 protein detection agent specifically binds Ang2 in a biological sample obtained from the subject (e.g., color reagents, stop solutions).

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. In case of conflict, the present application, including definitions, will control. The materials, methods, and examples are illustrative only and not intended to be limiting.

Other features and advantages of the invention will be apparent from the following detailed description, and from the claims.

### [Brief Description of Drawings]

[Fig. 1]

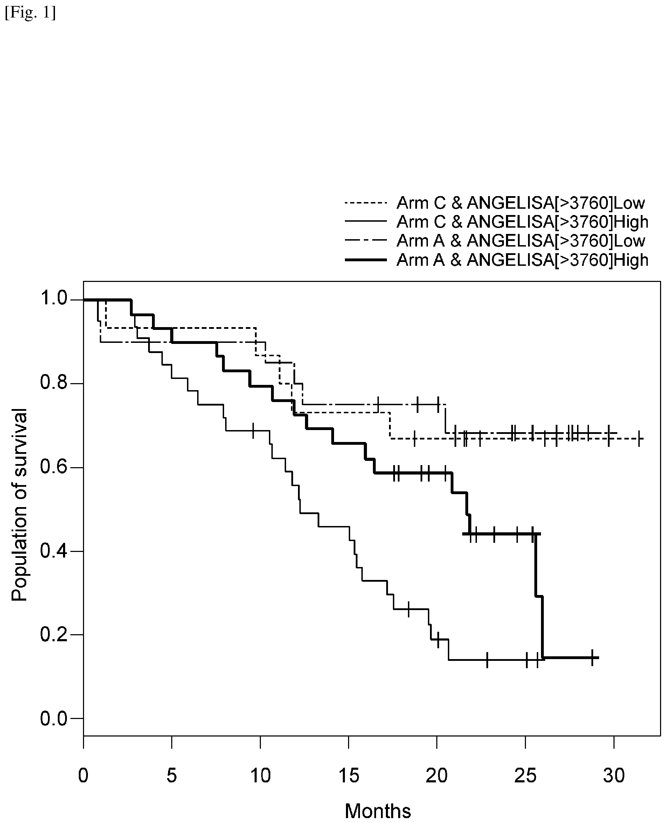

   Fig. 1 is Kaplan Meier curves of overall survival (OS) in combination and everolimus arms. In each arm, two curves are plotted based on Ang2 baseline level (> 3760 pg/ml or ≦ 3760 pg/ml). Thin curve in black: Ang2 high group in everolimus arm, bold curve in black: Ang2 high group in the combination arm, thin curve in gray: Ang2 low group in everolimus arm, and bold curve in gray: Ang2 low group in the combination arm.
[Fig. 2]

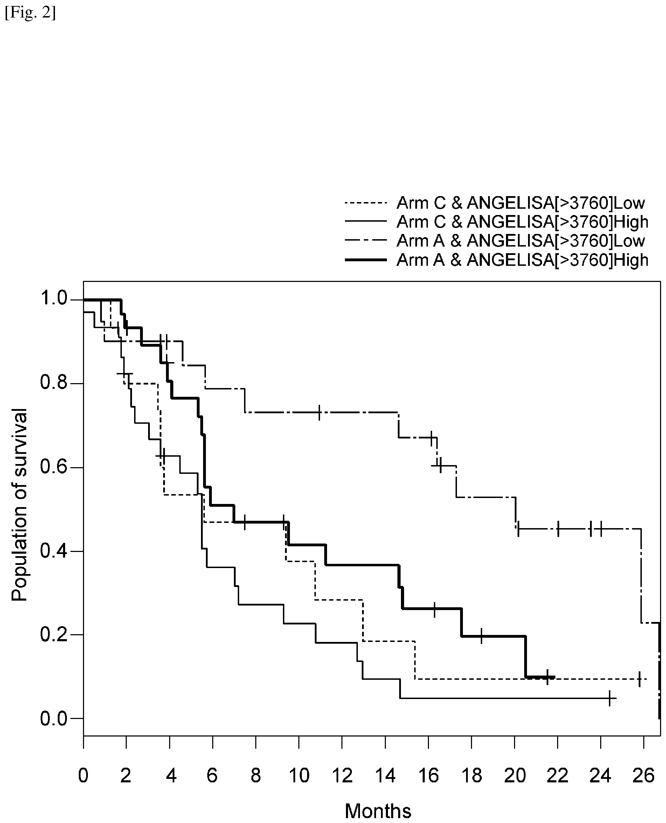

   Fig. 2 is Kaplan Meier curves of progression free survival (PFS) in combination and everolimus arms. In each arm, two curves are plotted based on Ang2 baseline level (> 3760 pg/ml or ≦ 3760 pg/ml). Thin curve in black: Ang2 high group in everolimus arm, bold curve in black: Ang2 high group in the combination arm, thin curve in gray: Ang2 low group in everolimus arm, and bold curve in gray: Ang2 low group in the combination arm.

### [Description of Embodiments]

This disclosure provides methods and compositions for identifying a renal cell carcinoma subject (such as a human patient) in need of a combination therapy comprising lenvatinib or a pharmaceutically acceptable salt thereof (e.g., lenvatinib mesylate) and everolimus. The disclosure provides Ang2 as a predictive biomarker to identify those subjects having, suspected of having, or at risk of developing, renal cell carcinoma (e.g., advanced or metastatic renal cell carcinoma) for whom administering a combination therapy comprising lenvatinib or a pharmaceutically acceptable salt thereof (e.g., lenvatinib mesylate) and everolimus is recommended. The biomarkers, compositions, and methods described herein are useful in selecting appropriate therapeutic modalities (e.g., combination therapy comprising lenvatinib or a pharmaceutically acceptable salt thereof (e.g., lenvatinib mesylate) and everolimus or an alternative renal cell carcinoma therapy) for subjects suffering from, suspected of having or at risk of developing renal cell carcinoma. Furthermore, this application provides methods of selecting patients having, suspected of having, or at risk of developing, renal cell carcinoma that could benefit from a combination therapy comprising lenvatinib or a pharmaceutically acceptable salt thereof (e.g., lenvatinib mesylate) and everolimus as well as methods of treatment.

### Definitions

The term "subject" means a mammal, including but not limited to, a human, a chimpanzee, an orangutan, a gorilla, a baboon, a monkey, a mouse, a rat, a pig, a horse, a dog, and a cow.

The term "a human subject ... in need of a combination therapy" means a human subject for whom a combination therapy is recommendable as a preferable treatment. Non-limiting examples of situation where a combination therapy is recommendable as a preferable treatment are: a combination therapy comprising two particular drugs is predicted as more effective than other combination therapy(s) comprising two drugs at least one of which are different from said two particular drugs; a combination therapy comprising two particular drugs is predicted as more effective than monotherapy with one of, or each of, said two drugs.

The term "combination therapy" means a therapy that concomitantly administers two or more drugs to a patient. The two or more drugs can be administered simultaneously, substantially simultaneously, or sequentially. In some cases, the two or more drugs may be formulated together (e.g., into a single tablet or capsule). In other cases, the two or more drugs are not co-formulated (e.g., they are administered as separate tablets or capsules).

The term "lenvatinib" refers to 4-(3-chloro-4-(cyclopropylaminocarbonyl)aminophenoxy)-7-methoxy-6-quinolinecarboxamide. This compound is disclosed in Example 368 (see, column 270) of U.S. Patent No. 7,253,286. U.S. Patent No. 7,253,286. The term "lenvatinib compound" refers to "lenvatinib or a pharmaceutically acceptable salt thereof." An example of a pharmaceutically acceptable salt of lenvatinib is lenvatinib mesylate. Lenvatinib mesylate is also referred to as E7080. Lenvatinib mesylate has been approved as LENVIMA™ by the U.S. Food and Drug Administration for the treatment of patients with locally recurrent or metastatic, progressive, radioactive iodine-refractory differentiated thyroid cancer.

The term "pharmaceutically acceptable salt" is not particularly restricted as to the type of salt. Examples of such salts include, but are not limited to, inorganic acid addition salt such as hydrochloric acid salt, sulfuric acid salt, carbonic acid salt, bicarbonate salt, hydrobromic acid salt and hydriodic acid salt; organic carboxylic acid addition salt such as acetic acid salt, maleic acid salt, lactic acid salt, tartaric acid salt and trifluoroacetic acid salt; organic sulfonic acid addition salt such as methanesulfonic acid salt, hydroxymethanesulfonic acid salt, hydroxyethanesulfonic acid salt, benzenesulfonic acid salt, toluenesulfonic acid salt and taurine salt; amine addition salt such as trimethylamine salt, triethylamine salt, pyridine salt, procaine salt, picoline salt, dicyclohexylamine salt, N,N'-dibenzylethylenediamine salt, N-methylglucamine salt, diethanolamine salt, triethanolamine salt, tris(hydroxymethylamino)methane salt and phenethylbenzylamine salt; and amino acid addition salt such as arginine salt, lysine salt, serine salt, glycine salt, aspartic acid salt and glutamic acid salt. In one embodiment, the pharmaceutically acceptable salt is a methanesulfonic acid salt ("mesylate"). The methanesulfonic acid salt form (i.e., the mesylate) of 4-(3-chloro-4-(cyclopropylaminocarbonyl)aminophenoxy)-7-methoxy-6-quinolinecarboxamide is disclosed in US Patent 7,612,208.

The term "everolimus" refers to *(1R,9S, 12S, 15R,* 16E, 18R, 19*R*,21*R*,23*S*,24*E*,26*E*,28*E*,30*S*,32*S*,35*R*)-1,18- dihydroxy-12-{(1*R*)-2-[(1*S*,3*R*,4*R*)-4-(2-hydroxyethoxy)-3-methoxycyclohexyl]-1-methylethyl}-19,30-dimethoxy-15, 17,21,23,29,35-hexamethyl-11,3 6-dioxa-4-aza-tricyclo[30.3.1.0^{4,9}]hexatriaconta-16,24,26,28-tetraene-2,3, 10,14,20-pentaone. This compound is disclosed in U.S. Patent No. 5,665,772. U.S. Patent No. 5,665,772. Everolimus has been approved as AFINITOR (registered trademark) by U.S. Food and Drug Administration for the treatment of various diseases, including advanced renal cell carcinoma after failure of treatment with sunitinib or sorafenib.

The term "protein" means any peptide-linked chain of amino acids, regardless of length or post-translational modification. Typically, a protein described herein is "isolated" when it constitutes at least 60%, by weight, of the total protein in a preparation, e.g., 60% of the total protein in a sample. In some aspects, a protein described herein consists of at least 75%, at least 90%, or at least 99%, by weight, of the total protein in a preparation.

The term "VEGF-targeted therapy" means any therapy comprising an administration of an anti-tumor agent that acts on vascular endothelial growth factor (VEGF) receptor(s). Non-limiting examples of anti-tumor agents used for VEGF-targeted therapy are: sunitinib, sorafenib, pazopanib, bevacizumab, axitinib, vatalanib and tivozanib.

The term "responds/responsive to a therapy" means that the subject administered with the therapy shows a positive response to the therapy provided. Non-limiting examples of such a positive response are: a decrease in tumor size, a decrease in metastasis of a tumor, or an increased period of survival after treatment.

The term "baseline level" of a protein in a sample from a subject means the concentration of that protein in the sample before administration of the subject with a combination therapy of everolimus and lenvatinib or a pharmaceutically acceptable salt thereof.

### Angiopoietin 2

Angiopoietins are protein growth factors that promote angiogenesis (the formation of blood vessels from pre-existing blood vessels) and maturation of tumor blood vessels. Mouse knock out studies have shown that Angiopoietin 2 (Ang2) is required for the formation of mature blood vessels. Expression of Ang2 in the endothelial cells is sufficient to recruit myeloid cells and induce inflammation even in the absence of preceding proinflammatory stimuli.

Gene ID, related URL, protein ID and UniProtKB Accession No. of Ang2 are as follows:
Official Gene Symbol: Ang2
Gene ID: 285
URL: www.ncbi.nlm.nih.gov/gene/285
Alternative Symbols: Ang-2, ANG-2, ANG2, Ang2 (90), Ang290, ANGPT2
UniProtKB Accession No.: 015123

A high baseline level (e.g., protein or mRNA expression) compared to a control of Ang2 is indicative/predictive that a subject is in need of a combination therapy comprising a lenvatinib compound (e.g., lenvatinib mesylate) and everolimus. For example, high concentrations (compared to a control) of baseline Ang2 protein in a biological sample obtained from a subject prior to treatment with the therapy are predictive that the subject is in need of a combination therapy comprising a lenvatinib compound (e.g., lenvatinib mesylate) and everolimus.

In certain embodiments, a subject is determined to respond to a combination therapy comprising a lenvatinib compound (e.g., lenvatinib mesylate) and everolimus, if the subject shows a partial response following treatment with the therapy. "Partial Response" means at least 30% decrease in the sum of the longest diameter (LD) of target lesions, taking as reference the baseline summed LD. In some embodiments, a subject is determined to respond to a combination therapy comprising a lenvatinib compound and everolimus, if the subject shows tumor shrinkage post-treatment with the therapy. "% of maximum tumor shrinkage" (MTS) means percent change of sum of diameters of target lesions, taking as reference the baseline sum diameters. In other embodiments, a subject is determined to respond to a combination therapy comprising a lenvatinib compound and everolimus, if the subject shows overall survival. "Overall Survival" (OS) refers to the time from randomization until death from any cause. "Randomization" means randomization of a patient into a test group or a control group when therapy plan for a patient is determined. In some embodiments, a subject is determined to respond to a combination therapy comprising a lenvatinib compound and everolimus, if the subject shows both overall survival and tumor shrinkage. In other embodiments, a subject is determined to respond to a combination therapy comprising a lenvatinib compound and everolimus, if the subject shows progression free survival. "Progression Free Survival" (PFS) refers to the time from the date of randomization to the date of first documentation of disease progression or death, whichever occurs first. In some embodiments, a subject is determined to respond to a combination therapy comprising a lenvatinib compound and everolimus, if the subject shows both progression free survival and tumor shrinkage.

This disclosure provides methods of identifying a subject having renal cell carcinoma who is more likely to have survival benefits (e.g., overall survival (OS)) following a combination therapy comprising a lenvatinib compound (e.g., lenvatinib mesylate) and everolimus than a monotherapy comprising everolimus only. In this method, a biological sample of the subject, obtained prior to treatment with the combination therapy comprising a lenvatinib compound and everolimus, is assayed and the level of Ang2 protein is measured. A high concentration of the baseline Ang2 protein compared to a control indicates that the subject will more likely have survival benefits (e.g., OS) following combination therapy comprising a lenvatinib compound and everolimus than a monotherapy comprising everolimus only.

In certain aspects, lenvatinib compound (e.g., lenvatinib mesylate) can be administered to a subject of the present invention orally once daily at a dosage of 12, 18 or 24 mg (each calculated as lenvatinib free base).

In certain aspects, everolimus can be administered to a subject of the present invention orally once daily at a dosage of 5 or 10 mg.

The concentration of Ang2 can be measured using any method known in the art such as an immunological assay. Non-limiting examples of such methods include enzyme immunoassay, radioimmunoassay, chemiluminescent immunoassay, electrochemiluminescence immunoassay, latex turbidimetric immunoassay, latex photometric immunoassay, immuno-chromatographic assay, and western blotting. In certain embodiments, the concentration of Ang2 is measured by enzyme immunoassay.

### Controls

As described above, the methods of the present invention can involve, measuring the baseline level of Ang2 in a biological sample from a subject having, suspected of having or at risk of developing renal cell carcinoma, wherein the expression level of Ang2, compared to a control, predicts that the subject is in need of (or can benefit from) a combination treatment comprising a lenvatinib compound (e.g., lenvatinib mesylate) and everolimus. In certain embodiments, when the concentration of baseline Ang2 in a biological sample from a subject having, suspected of having or at risk of developing renal cell carcinoma is higher than the control, the subject is identified as in need of a combination therapy comprising a lenvatinib compound and everolimus. In this context, the term "control" includes a sample (e.g., from the same tissue) obtained from a subject who is not in need of a combination therapy comprising a lenvatinib compound (e.g., lenvatinib mesylate) and everolimus. Such subject who is not in need of a combination therapy comprising a lenvatinib compound (e.g., lenvatinib mesylate) and everolimus may include a subject who is predicted to respond to the combination therapy but such response to the combination therapy is not significantly better than a predicted response to a monotherapy with everolimus. The term "control" also includes a sample (e.g., from the same tissue) obtained in the past from a subject who is known to be not in need of a combination therapy comprising a lenvatinib compound and everolimus and used as a reference for future comparisons to test samples taken from subjects for which necessity for the combination therapy is to be predicted.

In some embodiments, a "positive control" may be used instead of a "control." The "positive control" concentration for Ang2 in a particular cell type or tissue may alternatively be pre-established by an analysis of one or more subjects that have been identified as in need of a combination therapy comprising lenvatinib compound (e.g., lenvatinib mesylate) and everolimus. This pre-established reference value (which may be an average or median expression level taken from multiple subjects that have been identified as in need of a combination therapy) may then be used as the "positive control" expression level in the comparison with the test sample. In such a comparison, the subject is predicted to be in need of a combination therapy comprising a lenvatinib compound (e.g., lenvatinib mesylate) and everolimus if the concentration of Ang2 being analyzed is the same as, or comparable to (at least 85% but less than 100% of), the pre-established positive control reference.

In certain embodiments, the "control" is a pre-determined cut-off value.

### Cut-Off Values

In some embodiments, the methods described herein include determining if the concentration of Ang2 falls above or below a predetermined cut-off value.

In accordance with the methods and compositions described herein, a reference concentration of baseline Ang2 is identified as a cut-off value, above or below of which is predictive of necessity for a combination therapy comprising a lenvatinib compound (e.g., lenvatinib mesylate) and everolimus. Some cut-off values are not absolute in that clinical correlations can still remain significant over a range of values on either side of the cutoff; however, it is possible to select an optimal cut-off value (e.g. varying H-scores) of concentration of Ang2 for a particular sample type. Cut-off values determined for use in the methods described herein can be compared with, e.g., published ranges of concentrations but can be individualized to the methodology used and patient population. It is understood that improvements in optimal cut-off values could be determined depending on the sophistication of statistical methods used and on the number and source of samples used to determine reference level values for the different sample types. Therefore, established cut-off values can be adjusted up or down, on the basis of periodic re-evaluations or changes in methodology or population distribution.

The reference concentration of Ang2 can be determined by a variety of methods. The reference level can be determined by comparison of the concentration of Ang2 of interest in, e.g., populations of subjects (e.g., patients) that are in need of a combination therapy comprising a lenvatinib compound (e.g., lenvatinib mesylate) and everolimus or not in need of a combination therapy comprising a lenvatinib compound and everolimus. This can be accomplished, for example, by histogram analysis, in which an entire cohort of patients are graphically presented, wherein a first axis represents the concentration of Ang2 and a second axis represents the number of subjects in the cohort whose sample contain one or more concentrations. Determination of the reference concentration of Ang2 can then be made based on an amount or concentration which best distinguishes these separate groups. The reference level can be a single number, equally applicable to every subject, or the reference level can vary, according to specific subpopulations of subjects. For example, older subjects can have a different reference level than younger subjects for the same cancer. In addition, a subject with more advanced disease (e.g., an advanced or metastatic renal cell carcinoma) can have a different reference value than one with a milder form of the disease.

The pre-established cut-off value can be a protein concentration that is determined based on receiver operating characteristic (ROC) analysis. ROC curves are used to determine a cut-off value for a clinical test. Consider the situation where there are two groups of patients and by using an established standard technique one group is known to be in need of a combination therapy comprising lenvatinib compound and everolimus, and the other is known to not in need of a combination therapy comprising lenvatinib compound and everolimus. A measurement using a biological sample from all members of the two groups is used to test for the necessity for a combination therapy comprising lenvatinib compound and everolimus. The test will find some, but not all, subjects that are in need of a combination therapy comprising lenvatinib compound and everolimus. The ratio of the subjects in need of the combination therapy found by the test to the total number of the subjects in need of the combination therapy (known by the established standard technique) is the true positive rate (also known as sensitivity). The test will find some, but not all, the subjects not in need of a combination therapy comprising a lenvatinib compound and everolimus. The ratio of the subjects not in need of the combination therapy found by the test to the total number of the subjects not in need of the combination therapy (known by the established standard technique) is the true negative rate (also known as specificity). The hope is that the ROC curve analysis of the test above will find a cut-off value that will minimize the number of false positives and false negatives. A ROC is a graphical plot which illustrates the performance of a binary class stratifier system as its discrimination threshold is varied. It is created by plotting the fraction of true positives out of the positives versus the fraction of false positives out of the negatives, at various threshold settings.

In one embodiment, the concentration of Ang2 is determined based on ROC analysis predicting tumor response with a positive predictive value, wherein a concentration of Ang2 equal to or below the pre-established cut-off value is a low concentration of Ang2 and a value higher than the pre-established cut-off value is a high concentration of Ang2. The positive predictive value is the proportion of positive test results that are true positives; it reflects the probability that a positive test reflects the underlying condition being tested for. Methods of constructing ROC curves and determining positive predictive values are well known in the art. In certain embodiments, tumor response is an objective response rate (ORR), a clinical benefit rate (CBR) or % of maximum tumor shrinkage.

In another embodiment, the pre-established cut-off value can be a concentration of Ang2 that is determined based on simulation models predicting survival, and wherein a concentration of Ang2 equal to or below the pre-established cut-off value is a low concentration of Ang2 and a value higher than the pre-established cut-off value is a high concentration of Ang2. In some embodiments, survival is progression free survival (PFS). In other embodiments, survival is overall survival (OS).

In certain embodiments, the pre-established cut-off value for Ang2 protein is within a concentration range of 3565 to 5650 pg/ml. In a specific embodiment, the pre-established cut-off value for Ang2 protein is about 3760 pg/ml. In all of these embodiments, a concentration of Ang2 protein equal to or below the pre-established cut-off value is a low concentration of Ang2 and a value higher than the pre-established cut-off value is a high concentration of Ang2. In this context "about" means ±10%.

### Biological Samples

Suitable biological samples for the methods described herein include any biological fluid, cell, tissue, or fraction thereof, which contains Ang2 protein. A biological sample can be, for example, a specimen obtained from a subject (e.g., a mammal such as a human) or can be derived from such a subject. For example, a sample can be a tissue section obtained by biopsy, archived tumor tissue, or cells that are placed in or adapted to tissue culture. A biological sample can also be a biological fluid such as blood, plasma, serum, or such a sample absorbed onto a substrate (e.g., glass, polymer, paper). A biological sample can also include a renal cell carcinoma tissue sample. In specific embodiments, the biological sample is a tumor cell(s) or a tumor tissue obtained from a region of the subject suspected of containing a tumor or a pre-cancerous lesion. For example, the biological sample may be a renal cell carcinoma tumor sample. A biological sample can be further fractionated, if desired, to a fraction containing particular cell types. For example, a blood sample can be fractionated into serum or into fractions containing particular types of blood cells such as red blood cells or white blood cells (leukocytes). If desired, a sample can be a combination of samples from a subject such as a combination of a tissue and fluid sample.

The biological samples can be obtained from a subject having, suspected of having, or at risk of developing, a renal cell carcinoma. In certain embodiments, the subject has advanced or metastatic renal cell carcinoma. In some embodiments, the subject has recurrent renal cell carcinoma. In other embodiments, the subject has an unresectable advanced or metastatic renal cell carcinoma. In other aspects, the subject has a stage III renal cell carcinoma. In certain aspects, the subject has a stage IV renal cell carcinoma.

Any suitable methods for obtaining the biological samples can be employed, although exemplary methods include, e.g., phlebotomy, fine needle aspirate biopsy procedure. Samples can also be collected, e.g., by microdissection (e.g., laser capture microdissection (LCM) or laser microdissection (LMD)).

Methods for obtaining and/or storing samples that preserve the activity or integrity of molecules (e.g., nucleic acids or proteins) in the sample are well known to those skilled in the art. For example, a biological sample can be further contacted with one or more additional agents such as buffers and/or inhibitors, including one or more of nuclease, protease, and phosphatase inhibitors, which preserve or minimize changes in the molecules (e.g., nucleic acids or proteins) in the sample. Such inhibitors include, for example, chelators such as ethylenediamine tetraacetic acid (EDTA), ethylene glycol bis(P-aminoethyl ether) N,N,N1,N1-tetraacetic acid (EGTA), protease inhibitors such as phenylmethylsulfonyl fluoride (PMSF), aprotinin, leupeptin, antipain, and the like, and phosphatase inhibitors such as phosphate, sodium fluoride, vanadate, and the like. Suitable buffers and conditions for isolating molecules are well known to those skilled in the art and can be varied depending, for example, on the type of molecule in the sample to be characterized (see, for example, Ausubel et al. Current Protocols in Molecular Biology (Supplement 47), John Wiley & Sons, New York (1999); Harlow and Lane, Antibodies: A Laboratory Manual (Cold Spring Harbor Laboratory Press (1988); Harlow and Lane, Using Antibodies: A Laboratory Manual, Cold Spring Harbor Press (1999); Tietz Textbook of Clinical Chemistry, 3rd ed. Burtis and Ashwood, eds. W.B. Saunders, Philadelphia, (1999)). A sample also can be processed to eliminate or minimize the presence of interfering substances. For example, a biological sample can be fractionated or purified to remove one or more materials that are not of interest. Methods of fractionating or purifying a biological sample include, but are not limited to, chromatographic methods such as liquid chromatography, ion-exchange chromatography, size-exclusion chromatography, or affinity chromatography. For use in the methods described herein, a sample can be in a variety of physical states. For example, a sample can be a liquid or solid, can be dissolved or suspended in a liquid, can be in an emulsion or gel, or can be absorbed onto a material.

### Determining Expression Levels/Concentrations of Biomarkers

Gene expression can be detected as, e.g., protein or RNA expression of a target gene. That is, the presence or expression level (amount) of a gene can be determined by detecting and/or measuring the level of mRNA or protein expression of the gene. In some embodiments, expression of Ang2 can be detected as the activity of Ang2 encoded by Ang2 gene.

In one embodiment, the expression of Ang2 can be determined by detecting and/or measuring expression or concentration of Ang2 encoded by the Ang2 gene. Methods of determining protein expression/concentration are well known in the art. A generally used method involves the use of antibodies specific for the target protein of interest. For example, methods of determining protein expression include, but are not limited to, western blot or dot blot analysis, immunohistochemistry (e.g., quantitative immunohistochemistry), immunocytochemistry, enzyme-linked immunosorbent assay (ELISA), enzyme-linked immunosorbent spot (ELISPOT; Coligan, J. E., et al., eds. (1995) Current Protocols in Immunology. Wiley, New York), radioimmunoassay, chemiluminescent immunoassay, electrochemiluminescence immunoassay, latex turbidimetric immunoassay, latex photometric immunoassay, immuno-chromatographic assay, and antibody array analysis (see, e.g., U.S. Publication Nos. 20030013208 and 2004171068). Further description of many of the methods above and additional methods for detecting protein expression can be found in, e.g., Sambrook et al. (supra).

In one example, the presence or amount of expression of Ang2 can be determined using a western blotting technique. For example, a lysate can be prepared from a biological sample, or the biological sample itself, can be contacted with Laemmli buffer and subjected to sodium-dodecyl sulfate polyacrylamide gel electrophoresis (SDS-PAGE). SDS-PAGE-resolved proteins, separated by size, can then be transferred to a filter membrane (e.g., nitrocellulose) and subjected to immunoblotting techniques using a detectably-labeled antibody specific to Ang2. The presence or amount of bound detectably-labeled antibody indicates the presence or amount of protein in the biological sample.

In another example, an immunoassay can be used for detecting and/or measuring the protein expression of Ang2. As above, for the purposes of detection, an immunoassay can be performed with an antibody that bears a detection moiety (e.g., a fluorescent agent or enzyme). Proteins from a biological sample can be conjugated directly to a solid-phase matrix (e.g., a multi-well assay plate, nitrocellulose, agarose, sepharose, encoded particles, or magnetic beads) or it can be conjugated to a first member of a specific binding pair (e.g., biotin or streptavidin) that attaches to a solid-phase matrix upon binding to a second member of the specific binding pair (e.g., streptavidin or biotin). Such attachment to a solid-phase matrix allows the proteins to be purified away from other interfering or irrelevant components of the biological sample prior to contact with the detection antibody and also allows for subsequent washing of unbound antibody. Here as above, the presence or amount of bound detectably-labeled antibody indicates the presence or amount of protein in the biological sample.

There is no particular restriction as to the form of the antibody and the present disclosure includes polyclonal antibodies, as well as monoclonal antibodies. The antiserum obtained by immunizing animals, such as rabbits with Ang2 or fragment thereof, as well polyclonal and monoclonal antibodies of all classes, human antibodies, and humanized antibodies produced by genetic recombination, are also included.

An intact protein or its partial peptide may be used as the antigen for immunization. As partial peptides of the proteins, for example, the amino (N)-terminal fragment of the protein and the carboxy (C)-terminal fragment can be given.

A gene encoding Ang2 or a fragment thereof (e.g., an immunological fragment) is inserted into a known expression vector, and, by transforming the host cells with the vector described herein, the desired protein or a fragment thereof is recovered from outside or inside the host cells using standard methods. This protein can be used as the sensitizing antigen. Also, cells expressing the protein, cell lysates, or a chemically synthesized protein of the invention may be also used as a sensitizing antigen.

The mammal that is immunized by the sensitizing antigen is not restricted; however, it is preferable to select animals by considering the compatibility with the parent cells used in cell fusion. Generally, animals belonging to the orders rodentia, lagomorpha, or primates are used. Examples of animals belonging to the order of rodentia that may be used include, for example, mice, rats, and hamsters. Examples of animals belonging to the order of lagomorpha that may be used include, for example, rabbits. Examples of animals belonging to the order of primates that may be used include, for example, monkeys. Examples of monkeys to be used include the infraorder catarrhini (old world monkeys), for example, *Macaca fascicularis*, rhesus monkeys, sacred baboons, and chimpanzees.

Moreover, the antibody of the present disclosure may be an antibody fragment or modified-antibody, so long as it binds to Ang2. For instance, Fab, F (ab') 2, Fv, or single chain Fv (scFv) in which the H chain Fv and the L chain Fv are suitably linked by a linker (Huston et al., Proc. Natl. Acad. Sci. USA, 85:5879-5883, (1988)) can be given as antibody fragments.

The antibodies may be conjugated to various molecules, such as fluorescent substances, radioactive substances, and luminescent substances. Methods to attach such moieties to an antibody are already established and conventional in the field (see, e.g., US 5,057,313 and 5,156,840).

Examples of methods that assay the antigen-binding activity of the antibodies include, for example, measurement of absorbance, enzyme-linked immunosorbent assay (ELISA), enzyme immunoassay (EIA), radioimmunoassay (RIA), and/or immunofluorescence. For example, when using ELISA, a protein encoded by a biomarker of the invention is added to a plate coated with the antibodies of the present disclosure, and then, the antibody sample, for example, culture supernatants of antibody-producing cells, or purified antibodies are added. Then, secondary antibody recognizing the primary antibody, which is labeled by alkaline phosphatase and such enzymes, is added, the plate is incubated and washed, and the absorbance is measured to evaluate the antigen-binding activity after adding an enzyme substrate such as p-nitrophenyl phosphate. As the protein, a protein fragment, for example, a fragment comprising a C-terminus, or a fragment comprising an N-terminus may be used. To evaluate the activity of the antibody of the invention, BIAcore (GE Healthcare) may be used.

By using these methods, the antibody of the invention and a sample presumed to contain Ang2 are contacted, and Ang2 is detected or assayed by detecting or assaying the immune complex formed between the above-mentioned antibody and the protein.

Mass spectrometry based quantitation assay methods, for example, but not limited to, multiple reaction monitoring (MRM)-based approaches in combination with stable-isotope labeled internal standards, are an alternative to immunoassays for quantitative measurement of proteins. These approaches do not require the use of antibodies and so the analysis can be performed in a cost- and time- efficient manner (see, for example, Addona et al., Nat. Biotechnol., 27:633-641, 2009; Kuzyk et al., Mol. Cell Proteomics, 8:1860-1877, 2009; Paulovich et al., Proteomics Clin. Appl., 2:1386-1402, 2008). In addition, MRM offers superior multiplexing capabilities, allowing for the simultaneous quantification of numerous proteins in parallel. The basic theory of these methods has been well-established and widely utilized for drug metabolism and pharmacokinetics analysis of small molecules.

### Creating A Response Profile

The methods described herein can also be used to generate a response profile for a subject having renal cell carcinoma about a combination therapy comprising lenvatinib compound (e.g., lenvatinib mesylate) and everolimus. The profile can include, e.g., information that indicates the baseline expression level of Ang2 before the treatment with lenvatinib or a pharmaceutically acceptable salt thereof; and/or the histological analysis of any renal cell carcinoma. The resultant information (lenvatinib therapy response profile) can be used for predicting that a subject (e.g., a human patient) having, suspected of having or at risk of developing renal cell carcinoma is in need of a combination therapy comprising a lenvatinib compound (e.g., lenvatinib mesylate) and everolimus.

It is understood that a lenvatinib compound (e.g., lenvatinib mesylate) response profile can be in electronic form (e.g., an electronic patient record stored on a computer or other electronic (computer-readable) media such as a DVD, CD, or floppy disk) or written form. The lenvatinib compound (e.g., lenvatinib mesylate) response profile can also include information for several (e.g., two, three, four, five, 10, 20, 30, 50, or 100 or more) subjects (e.g., human patients). Such multi-subject response profiles can be used, e.g., in analyses (e.g., statistical analyses) of particular characteristics of subject cohorts.

Responsiveness of a subject to a combination therapy comprising lenvatinib or a pharmaceutically acceptable salt thereof (e.g., lenvatinib mesylate) and everolimus can be classified in several ways and classification is dependent on the subject's disease, the severity of the disease, and the particular medicament the subject is administered. In the simplest sense, responsiveness is any decrease in the disease state as compared to pre-treatment, and non-responsiveness is the lack of any change in the disease state as compared to pre-treatment. Responsiveness of a subject (e.g., a human) with a renal cell carcinoma can be classified based on one or more of a number of objective clinical indicia such as, but not limited to, tumor size, Clinical Benefit (CB), Progression Free Survival (PFS), Overall Survival (OS), % of maximum tumor Shrinkage (MTS), or Objective Response Rate (ORR).

"Clinical benefit" refers to having one of the following statuses - Complete Response (CR), Partial Response (PR); or Stable Disease (SD) with 6 months or more progression free survival (PFS). "Complete Response" means complete disappearance of all target lesions. "Partial Response" means at least 30% decrease in the sum of the longest diameter (LD) of target lesions, taking as reference the baseline summed LD. "Progressive Disease" (PD) means at least 20% increase in the sum of the LD of target lesions, taking as reference the smallest summed LD recorded since the treatment started, or the appearance of one or more new lesions. "Stable Disease" means neither sufficient shrinkage of the target lesions to qualify for PR nor sufficient increase to qualify for progressive disease (PD), taking as reference the smallest summed LD since the treatment started.

"Overall Survival" (OS) is defined as the time from randomization until death from any cause. "Randomization" means randomization of a patient into a test group or a control group when therapy plan for a patient is determined.

"Progression Free Survival" (PFS) refers to the time from the date of randomization to the date of first documentation of disease progression or death, whichever occurs first.

"% of Maximum Tumor shrinkage" (MTS) means percent change of sum of diameters of target lesions, taking as reference the baseline sum diameters.

"Objective Response Rate " (ORR) compares subjects with either Complete Response (CR) or Partial Response (PR) with subjects with either Stable Disease (SD) or Progressive Disease (PD).

### Kits

This disclosure also provides kits. In certain aspects, the kit can include an antibody or antibodies that can be used to detect Ang2 or its concentration or expression levels. The antibodies in the kit may be monoclonal or polyclonal and can be further conjugated with a detectable label. The kits can, optionally, contain instructions for detecting and/or measuring the concentration of Ang2 in a biological sample.

The kits can optionally include, e.g., a control (e.g., a concentration standard for Ang2 protein). In some instances, the control can be an insert (e.g., a paper insert or electronic medium such as a CD, DVD, or floppy disk) containing an expression level or expression level ranges of Ang2 predictive of a necessity for a combination therapy comprising a lenvatinib compound (e.g., lenvatinib mesylate) and everolimus.

In some aspects, the kits can include one or more reagents for processing a biological sample (e.g., calibration reagents, buffers, diluents, color reagents, reagents to stop a reaction). For example, a kit can include reagents for isolating a protein from a biological sample and/or reagents for detecting the presence and/or amount of Ang2 in a biological sample (e.g., an antibody that binds to Ang2 and/or an antibody that binds the antibody that binds to Ang2).

In certain aspects, the kit includes at least one microplate (e.g., a 96 well plate; i.e., 12 strips of 8 wells). The microplate can be provided with its corresponding plate cover. The microplate can be polystyrene or of any other suitable material. The microplate can have the antibody that is used to identify the presence of Ang2 coated inside each well. The antibody may be conjugated to a detectable label. The kit may also include at least one adhesive strip.

In some aspects, the kits can include a software package for analyzing the results of, e.g., expression profile or a microarray analysis.

The kits described herein can also, optionally, include instructions for administering a combination therapy comprising a lenvatinib compound and everolimus, where the concentration of Ang2 predicts that a subject having, suspected of having or at risk of developing renal cell carcinoma is in need of a combination therapy comprising a lenvatinib compound (e.g., lenvatinib mesylate) and everolimus.

### [Examples]

### Example 1: Biomarker for identifying a subgroup of renal cell carcinoma patients in need of a combination therapy with lenvatinib and everolimus

*Purpose:* A Phase 2 study for lenvatinib was performed in patients with metastatic renal cell carcinoma (RCC) following a VEGF-targeted therapy. The importance of angiogenesis in RCC highlights the need to understand clinical mechanisms of escape from anti-angiogenic therapy, such as everolimus. This clinical biomarker analysis was conducted to identify predictive markers of clinical benefit in RCC patients upon the combination therapy with lenvatinib and everolimus compared to everolimus monotherapy.

The purpose of this analysis was to measure Ang2 level in blood samples, such as serum, obtained from patients in clinical trials at both pre- and post-treatment with lenvatinib, everolimus or the combination thereof, and to identify blood biomarkers which can be used to predict whether patients will need the combination therapy.

*Materials and Methods:* Patients having metastatic renal cell carcinoma after a prior VEGF-targeted therapy received lenvatinib mesylate ("lenvatinib"), everolimus, or the combination thereof until disease progression or development of unmanageable toxicities. Patients received lenvatinib at a dose of 24 mg (as lenvatinib free base; the same shall apply hereinafter) oral once daily, everolimus at a dose of 10 mg oral once daily, or lenvatinib at 18 mg and everolimus at 5 mg oral once daily. 153 patients were treated and evaluated for efficacy and molecular correlative analysis. This study was a part of a Phase 2 multicenter study of lenvatinib (NCT01136733). Pre- and post-treatment serum samples were collected for biomarker analysis. Serum samples were collected on Cycle 1 Day 1 (pre-treatment), Cycle 2 Day 15, and Day 1 of every cycle from Cycle 2, and at off-treatment. Each Cycle consisted of 4 weeks (28 days). Ang2 detection enzyme immunoassay kit (EIDIA Co., Ltd.) was used for the quantification of serum Ang2 level. The association between the concentration at Cycle 1 Day 1 ("baseline level") of Ang2 and clinical outcomes, such as progression free survival (PFS) and overall survival (OS), was assessed. PFS is defined as the time from the date of randomization to the date of first documentation of disease progression or death, whichever occurs first. PFS was based on investigator review data using RECIST 1.1. OS is defined as the time from randomization until death from any cause. "Randomization" means randomization of a patient into a test group or a control group when therapy plan for a patient is determined.

*Results:* From 153 patient samples, at maximum samples from 147 patients were used for baseline analysis. The correlative analysis of baseline Ang2 levels with OS showed significant associations between lower baseline Ang2 levels and longer OS in combination therapy with lenvatinib and everolimus (HR 1.94, *P*=0.002), lenvatinib monotherapy (HR 2.12, *P*<0.001) and everolimus monotherapy (HR 1.65, *P*<0.001). However, in dichotomized analysis of RCC patients with high (> 3760 pg/ml) vs low (≦ 3760 pg/ml) baseline Ang2 subgroups (33^{rd} percentile cut-off), in RCC patients having higher baseline Ang2 level the combination therapy showed improved outcome (OS) compared with everolimus monotherapy arm (Hazard Ratio=0.43, p=0.008, median OS=21.7 months vs 12.2 months) (Figure 1). Consistent with OS analysis, in RCC patients with higher baseline Ang2 level the combination therapy also showed longer PFS compared with everolimus monotherapy (Hazard Ratio=0.53, p=0.043, median PFS=6.9 months vs 5.5 months) (Figure 2). In RCC patient having lower baseline Ang2 level the combination therapy didn't show a significant improvement with OS compared with everolimus monotherapy (Hazard Ratio=0.90, p=0.864, median OS: not reached for both arms) (Figure 1), though the combination therapy showed longer PFS compared with everolimus arm (Hazard Ratio=0.30, p=0.006, median PFS=20.1 months vs 5.6 months). When 25^{th} percentile cut-off (3565 pg/ml) or 70^{th} percentile cut-off (5650 pg/ml) was used for the dichotomized analysis of the same RCC patients group, in the patients having higher baseline Ang2 level the combination therapy still showed improved outcome (OS) compared with everolimus monotherapy arm (For 25^{th} percentile cut-off: Hazard Ratio=0.42, p=0.005, median OS=21.8 months vs 12.2 months. For 70^{th} percentile cut-off: Hazard Ratio=0.39, p=0.031, median OS=21.7 months vs 12.2 months).

### Embodiments

Embodiments of the invention are as follows:
(1) A method of identifying a human subject having, suspected of having, or at risk of developing, a renal cell carcinoma in need of a combination therapy comprising lenvatinib or a pharmaceutically acceptable salt thereof and everolimus, the method comprising:
   assaying a biological sample obtained from the human subject and determining that the baseline level of angiopoietin-2 (Ang2) protein in the biological sample is higher than a control; and
   identifying the human subject having a higher concentration of Ang2 protein in the biological sample than a control as in need of the combination therapy comprising lenvatinib or a pharmaceutically acceptable salt thereof and everolimus;
   wherein the baseline level of Ang2 protein is the concentration of Ang2 protein in the sample of the subject who has not been administered the combination therapy; and
   wherein the control is a sample obtained from a subject who is not in need of the combination therapy.
(2) The method of (1), wherein the biological sample is selected from the group consisting of a blood sample, a serum sample, a plasma sample, a renal cell carcinoma archived tumor sample, and a renal cell carcinoma biopsy sample.
(3) The method of (1) or (2), wherein the biological sample is a serum sample.
(4) The method of any one of (1) to (3), wherein the renal cell carcinoma is an advanced renal cell carcinoma or a metastatic renal cell carcinoma.
(5) The method of any one of (1) to (4), wherein the human subject having, suspected of having, or at risk of developing, a renal cell carcinoma has experienced at least one prior VEGF-targeted therapy.
(6) The method of any one of (1) to (5), wherein the control is a pre-established cut-off value.
(7) The method of (6), wherein the pre-established cut-off value is an Ang2 protein concentration that is determined based on receiver operating characteristic analysis predicting tumor response with a higher positive predictive value.
(8) The method of (7), wherein tumor response is an objective response rate, a clinical benefit rate or % of maximum tumor shrinkage of at least 30%.
(9) The method of (6), wherein the pre-established cut-off value is an Ang2 protein concentration that is determined based on predicting survival using simulation models to separate two groups divided by the cut-off.
(10) The method of (9), wherein survival is overall survival.
(11) The method of (6), wherein the pre-established cut-off value is an Ang2 protein concentration within the range from 3565 to 5650 pg/ml.
(12) The method of any one of (1) to (11), wherein the concentration of Ang2 protein is measured by an immunological method.
(13) The method of (12), wherein the immunological method is selected from the group consisting of enzyme immunoassay, radioimmunoassay, chemiluminescent immunoassay, electrochemiluminescence immunoassay, latex turbidimetric immunoassay, latex photometric immunoassay, immuno-chromatographic assay, and western blotting.
(14) The method of any one of (1) to (12), wherein the concentration of the protein is measured by enzyme immunoassay.
(15) The method of any one of (1) to (14), wherein the pharmaceutically acceptable salt of lenvatinib is lenvatinib mesylate.
(16) Lenvatinib or a pharmaceutically acceptable salt thereof for a concomitant use with everolimus in treating a renal cell carcinoma in a human subject, wherein the human subject is identified by the method of (1) as a subject that is in need of a combination therapy comprising lenvatinib or a pharmaceutically acceptable salt thereof and everolimus.

While the invention has been described in conjunction with the detailed description thereof, the foregoing description is intended to illustrate the invention, which is defined by the scope of the appended claims.

## Claims

1. A method of identifying a human subject having, suspected of having, or at risk of developing, a renal cell carcinoma in need of a combination therapy comprising lenvatinib or a pharmaceutically acceptable salt thereof and everolimus, the method comprising:
assaying a biological sample obtained from the human subject and determining that the baseline level of angiopoietin-2 (Ang2) protein in the biological sample is higher than a control; and
identifying the human subject having a higher concentration of Ang2 protein in the biological sample than a control as in need of the combination therapy comprising lenvatinib or a pharmaceutically acceptable salt thereof and everolimus;
wherein the baseline level of Ang2 protein is the concentration of Ang2 protein in the sample of the subject who has not been administered the combination therapy; and
wherein the control is a sample obtained from a subject who is not in need of the combination therapy.

2. The method of claim 1, wherein the biological sample is selected from the group consisting of a blood sample, a serum sample, a plasma sample, a renal cell carcinoma archived tumor sample, and a renal cell carcinoma biopsy sample.

3. The method of claim 1 or 2, wherein the biological sample is a serum sample.

4. The method of any one of claims 1 to 3, wherein the renal cell carcinoma is an advanced renal cell carcinoma or a metastatic renal cell carcinoma.

5. The method of any one of claims 1 to 4, wherein the human subject having, suspected of having, or at risk of developing, a renal cell carcinoma has experienced at least one prior VEGF-targeted therapy.

6. The method of any one of claims 1 to 5, wherein the control is a pre-established cut-off value.

7. The method of claim 6, wherein the pre-established cut-off value is an Ang2 protein concentration that is determined based on receiver operating characteristic analysis predicting tumor response with a higher positive predictive value compared to no cut-off.

8. The method of claim 7, wherein tumor response is an objective response rate, a clinical benefit rate or % of maximum tumor shrinkage of at least 30%.

9. The method of claim 6, wherein the pre-established cut-off value is an Ang2 protein concentration that is determined based on predicting survival using simulation models to separate two groups divided by the cut-off.

10. The method of claim 9, wherein survival is overall survival.

11. The method of claim 6, wherein the pre-established cut-off value is an Ang2 protein concentration within the range from 3565 to 5650 pg/ml.

12. The method of any one of claims 1 to 11, wherein the concentration of Ang2 protein is measured by an immunological method.

13. The method of claim 12, wherein the immunological method is selected from the group consisting of enzyme immunoassay, radioimmunoassay, chemiluminescent immunoassay, electrochemiluminescence immunoassay, latex turbidimetric immunoassay, latex photometric immunoassay, immuno-chromatographic assay, and western blotting.

14. The method of any one of claims 1 to 12, wherein the concentration of the protein is measured by enzyme immunoassay.

15. The method of any one of claims 1 to 14, wherein the pharmaceutically acceptable salt of lenvatinib is lenvatinib mesylate.

16. Lenvatinib or a pharmaceutically acceptable salt thereof for a concomitant use with everolimus in the treatment of a renal cell carcinoma in a human subject, wherein the human subject is identified by the method of claim 1 as a subject that is in need of a combination therapy comprising lenvatinib or a pharmaceutically acceptable salt thereof and everolimus.

## Patentansprüche

1. Verfahren zur Identifikation eines menschlichen Individuums, das ein Nierenzellkarzinom aufweist, bei dem vermutet wird, dass es ein Nierenzellkarzinom aufweist oder das ein Risiko aufweist, ein Nierenzellkarzinom zu entwickeln, das eine Kombinationstherapie benötigt, die Lenvatinib oder ein pharmazeutisch annehmbares Salz davon und Everolimus umfasst, das Verfahren umfassend:
Untersuchen einer biologischen Probe, die von dem menschlichen Individuum erhalten wurde, und Bestimmen, dass der Ausgangswert des Angiopoetin-2 (Ang2)-Proteins in der biologischen Probe höher ist als eine Kontrolle;
und Identifizieren des menschlichen Individuums mit einer höheren Konzentration des Ang-2-Proteins in der biologischen Probe als eine Kontrolle als eines, das die Kombinationstherapie umfassend Lenvatinib oder ein pharmazeutisch annehmbares Salz davon und Everolimus benötigt;
worin der Ausgangswert des Ang2-Proteins die Konzentration des Ang2-Proteins in der Probe des Individuums ist, dem die Kombinationstherapie nicht verabreicht wurde; und worin die Kontrolle eine Probe ist, die von einem Individuum erhalten wurde, das die Kombinationstherapie nicht benötigt.

2. Verfahren gemäß Anspruch 1, worin die biologische Probe ausgewählt ist aus der Gruppe, bestehend aus einer Blutprobe, einer Serumprobe, einer Plasmaprobe, einer archivierten Nierenzellkarzinom-Tumorprobe und einer Nierenzellkarzinom-Biopsieprobe.

3. Verfahren gemäß Anspruch 1 oder 2, worin die biologische Probe eine Serumprobe ist.

4. Verfahren gemäß einem beliebigen der Ansprüche 1 bis 3, worin das Nierenzellkarzinom ein fortgeschrittenes Nierenzellkarzinom oder ein metastasiertes Nierenzellkarzinom ist.

5. Verfahren gemäß einem beliebigen der Ansprüche 1 bis 4, worin das menschliche Individuum, das ein Nierenzellkarzinom aufweist, bei dem vermutet wird, dass es ein Nierenzellkarzinom aufweist oder das ein Risiko aufweist, ein Nierenzellkarzinom zu entwickeln, mindestens eine vorherige auf VEGF gerichtete Therapie erfahren hat.

6. Verfahren gemäß einem beliebigen der Ansprüche 1 bis 5, worin die Kontrolle ein vorher bestimmter Cut-off-Wert ist.

7. Verfahren gemäß Anspruch 6, worin der vorher bestimmte Cut-off-Wert eine Ang2-Proteinkonzentration ist, die auf Basis einer Receiver-operating-characteristics-Analyse bestimmt wird, die eine Tumorantwort mit einem höheren positiven Vorhersagewert als ohne Cut-off vorhersagt.

8. Verfahren gemäß Anspruch 7, worin die Tumorantwort eine objektive Ansprechrate, eine klinische Nutzenrate oder % des maximalen Tumorrückgangs von mindestens 30% ist.

9. Verfahren gemäß Anspruch 6, worin der vorher bestimmte Cut-off-Wert eine Ang2-Proteinkonzentration ist, die auf Basis der Vorhersage des Überlebens unter Verwendung von Simulationsmodellen bestimmt wird, um zwei durch den Cut-off unterteilte Gruppen zu trennen.

10. Verfahren gemäß Anspruch 9, worin das Überleben ein Gesamtüberleben ist.

11. Verfahren gemäß Anspruch 6, worin der vorher bestimmte Cut-off-Wert eine Ang2-Proteinkonzentration innerhalb des Bereichs von 3565 bis 5650 pg/ml ist.

12. Verfahren gemäß einem beliebigen der Ansprüche 1 bis 11, worin die Konzentration des Ang2-Proteins durch ein immunologisches Verfahren gemessen wird.

13. Verfahren gemäß Anspruch 12, worin das immunologische Verfahren ausgewählt ist aus der Gruppe, bestehend aus Enzymimmunoassay, Radioimmunoassay, Chemilumineszenz-Immunoassay, Elektrochemilumineszenz-Immunoassay, Latexturbidimetrischem Immunoassay, Latexphotometrischem Immunoassay, immunochromatographischem Assay und Western Blotting.

14. Verfahren gemäß einem beliebigen der Ansprüche 1 bis 12, worin die Proteinkonzentration durch einen Enzymimmunoassay gemessen wird.

15. Verfahren gemäß einem beliebigen der Ansprüche 1 bis 14, worin das pharmazeutisch annehmbare Salz von Lenvatinib Lenvatinibmesylat ist.

16. Lenvatinib oder ein pharmazeutisch annehmbares Salz davon zur gleichzeitigen Verwendung mit Everolimus bei der Behandlung eines Nierenzellkarzinoms in einem menschlichen Individuum, worin das menschliche Individuum mittels des Verfahrens gemäß Anspruch 1 als ein Individuum identifiziert wird, das eine Kombinationstherapie, umfassend Lenvatinib oder ein pharmazeutisch annehmbares Salz davon mit Everolimus, benötigt.

## Revendications

1. Procédé d'identification qu'un sujet humain présentant, suspecté de présenter, ou risquant de développer, un carcinome à cellules rénales a besoin d'une multithérapie comprenant du lenvatinib ou un sel pharmaceutiquement acceptable de celui-ci et de l'évérolimus, le procédé comprenant :
le dosage d'un échantillon biologique obtenu à partir du sujet humain et la détermination que le taux de base de la protéine angiopoïétine-2 (Ang2) dans l'échantillon biologique est supérieur à un témoin ; et
l'identification que le sujet humain présentant une concentration en protéine Ang2 dans l'échantillon biologique supérieure à un témoin a besoin de la multithérapie comprenant du lenvatinib ou un sel pharmaceutiquement acceptable de celui-ci et de l'évérolimus ;
dans lequel le taux de base de la protéine Ang2 est la concentration en protéine Ang2 dans l'échantillon du sujet qui n'a pas reçu la multithérapie ; et
dans lequel le témoin est un échantillon obtenu à partir d'un sujet qui n'a pas besoin de la multithérapie.

2. Procédé selon la revendication 1, dans lequel l'échantillon biologique est sélectionné dans le groupe consistant en un échantillon de sang, un échantillon de sérum, un échantillon de plasma, un échantillon de tumeur archivé en tant que carcinome à cellules rénales, et un échantillon de biopsie de carcinome à cellules rénales.

3. Procédé selon la revendication 1 ou 2, dans lequel l'échantillon biologique est un échantillon de sérum.

4. Procédé selon l'une quelconque des revendications 1 à 3, dans lequel le carcinome à cellules rénales est un carcinome à cellules rénales avancé ou un carcinome à cellules rénales métastatique.

5. Procédé selon l'une quelconque des revendications 1 à 4, dans lequel le sujet humain présentant, suspecté de présenter, ou risquant de développer, un carcinome à cellules rénales a subi au moins une thérapie antérieure ciblant VEGF.

6. Procédé selon l'une quelconque des revendications 1 à 5, dans lequel le témoin est une valeur seuil préétablie.

7. Procédé selon la revendication 6, dans lequel la valeur seuil préétablie est une concentration en protéine Ang2 qui est déterminée sur la base d'une analyse de caractéristique de fonctionnement de receveur prédisant une réponse tumorale avec une valeur prédictive positive plus élevée par rapport à l'absence de seuil.

8. Procédé selon la revendication 7, dans lequel la réponse tumorale est un taux de réponse objectif, un taux de bénéfice clinique ou un % de diminution maximale de la tumeur d'au moins 30 %.

9. Procédé selon la revendication 6, dans lequel la valeur seuil préétablie est une concentration en protéine Ang2 qui est déterminée sur la base d'une prédiction de survie en utilisant des modèles de simulation pour séparer deux groupes divisés par le seuil.

10. Procédé selon la revendication 9, dans lequel la survie est une survie globale.

11. Procédé selon la revendication 6, dans lequel la valeur seuil préétablie est une concentration en protéine Ang2 dans la plage allant de 3565 à 5650 pg/ml.

12. Procédé selon l'une quelconque des revendications 1 à 11, dans lequel la concentration en protéine Ang2 est mesurée par un procédé immunologique.

13. Procédé selon la revendication 12, dans lequel le procédé immunologique est sélectionné dans le groupe consistant en un dosage immuno-enzymatique, un dosage radio-immunologique, un dosage immunologique par chimioluminescence, un dosage immunologique par électrochimioluminescence, un dosage immunologique turbidimétrique sur latex, un dosage immunologique photométrique sur latex, un dosage immuno-chromatographique, et un transfert de Western.

14. Procédé selon l'une quelconque des revendications 1 à 12, dans lequel la concentration de la protéine est mesurée par un dosage immuno-enzymatique.

15. Procédé selon l'une quelconque des revendications 1 à 14, dans lequel le sel pharmaceutiquement acceptable du lenvatinib est le mésylate de lenvatinib.

16. Lenvatinib ou un sel pharmaceutiquement acceptable de celui-ci pour une utilisation concomitante avec l'évérolimus dans le traitement d'un carcinome à cellules rénales chez un sujet humain, dans lequel le sujet humain est identifié par le procédé selon la revendication 1 comme étant un sujet qui a besoin d'une multithérapie comprenant du lenvatinib ou un sel pharmaceutiquement acceptable de celui-ci et de l'évérolimus.
